# EUROPEAN PATENT APPLICATION

(11) **EP 3 779 854 A1**
(43) Date of publication of application: **17.02.2021**
(21) Application number: 18911557.9
(22) Date of filing: 07.12.2018
(51) Int. Cl.: G06Q 50/10, A61B 5/00

(54) **MANAGEMENT SYSTEM, MANAGEMENT METHOD, AND PROGRAM**

(30) Priority: 30.03.2018 JP 2018069450
(71) Applicant: Panasonic Intellectual Property Management Co., Ltd., Osaka-shi, Osaka 540-6207 (JP)
(72) Inventor: MIYAMOTO Shingo, Osaka-shi, Osaka 540-6207 (JP)
(74) Representative: Appelt, Christian W.
(86) International application number: PCT/JP2018/045139
(87) International publication number: WO 2019/187367

(57) **Abstract**

A first acquisition unit 310 acquires device identification information for identifying a device 100 and user identification information for identifying a user in a system that does not manage the device 100. A second acquisition unit 312 acquires device user identification information for identifying a user in the device 100 and the user identification information. A first management unit 316 maps the user identification information, the device identification information, and the device user identification information to each other and manages the information accordingly, by using the user identification information.

## Description

### [TECHNICAL FIELD]

The present disclosure relates to a management system, a management method, and a program.

### [BACKGROUND ART]

A biological information system is used to sense a user of a rest room facility. The biological information system is comprised of a biological information collecting means having a built-in individual identification means and of a biological information recording means. In this biological information system, individual identification results obtained by the individual identification means are shared within the biological information system, and biological information measured by all biological information collecting means located within the biological information system is linked to the user and recorded in the biological information recording means (see, for example, patent literature 1).

[Patent Literature 1] JP2000-139858

### [SUMMARY OF INVENTION]

### [TECHNICAL PROBLEM]

User identification information in an IoT (Internet of things) device such as a rest room facility is used to manage the IoT device. However, user identification information in the IoT device cannot be used in a system different from the system that manages the IoT device. This makes it impossible to coordinate a system different from the system that manages the IoT device with the IoT device. However, it is desirable to coordinate the systems for the purpose of improving user convenience.

The disclosure addresses the above-described issue, and a general purpose thereof is to provide a technology for coordinating a system different from the system that manages a device with the device.

### [SOLUTION TO PROBLEM]

A management system according to an embodiment of the present disclosure includes: a first acquisition unit that acquires device identification information for identifying a device and user identification information for identifying a user in a system that does not manage the device; a second acquisition unit that acquires device user identification information for identifying a user in the device and the user identification information; and a management unit that maps the user identification information, the device identification information, and the device user identification information to each other and manages the information accordingly, by using the user identification information.

Another embodiment of the present disclosure relates to a management method. The method includes: acquiring device identification information for identifying a device and user identification information for identifying a user in a system that does not manage the device; acquiring device user identification information for identifying a user in the device and the user identification information; and mapping the user identification information, the device identification information, and the device user identification information to each other and managing the information accordingly, by using the user identification information.

Optional combinations of the aforementioned constituting elements, and implementations of the present disclosure in the form of methods, systems, computer programs, recording mediums recording computer programs, etc. may also be practiced as additional modes of the present disclosure.

### [ADVANTAGEOUS EFFECTS OF INVENTION]

According to the present disclosure, it is possible to coordinate a system different from the system that manages a device with the device.

### [BRIEF DESCRIPTION OF DRAWINGS]

Fig. 1 shows a configuration of a communication system according to the embodiment;
Fig. 2 shows functional blocks of the communication system of Fig. 1;
Figs. 3A-3B show a data structure of data stored in the management unit of Fig. 2;
Figs. 4A-4B show a data structure of data stored in the management apparatus of Fig. 2;
Figs. 5A-5C show screens displayed on the display unit of Fig. 2;
Fig. 6 shows a data structure of data stored in the storage unit of Fig. 2;
Fig. 7 is a flowchart showing a sequence of steps performed by the management apparatus of Fig. 2; and
Fig. 8 is a flowchart showing another sequence of steps performed by the management apparatus of Fig. 2.

### [DESCRIPTION OF EMBODIMENTS]

The knowledge that provides the basis of an embodiment of the present disclosure will be discussed before discussing the embodiment in specific details. The embodiment relates to a communication system configured to link multiple types of identification information on a user and manage the information accordingly. In an IoT device provided in a residence, etc. (hereinafter, "device"), identification information on users (hereinafter, "device user identification information") is defined, and user-specific settings are applied based on the device user identification information. Since the device is provided in a residence, etc., the number of items of device user identification information is about as many as the total number of users in the residence (e.g., 4). Identification information on users (hereinafter, "user identification information") is also defined in a system (hereinafter, "second system") different from the system (hereinafter, "first system") that manages the device as described above. For example, the second system is a system that provides e-commerce service on the Internet. Therefore, the number of items of user identification information is, for example, 10,000 or larger. Thus, the total number of items of user identification information is larger than the total number of items of device user identification information.

Generally, the device user identification information can identify users in the residence and is used on within the device or within the first system. Services that use the device user identification information are limited. Meanwhile, the user identification information can identify users using the Internet and so can be used not only in a single e-commerce transaction but also in multiple types of e-commerce transactions and services other than e-commerce. Therefore, there are a variety of services that use the user identification information. By mapping the device user identification information with the user identification information in this situation, it will be possible to use a variety of services in the device or in the first system. However, since the total number of items of user identification information is larger than the total number of items of device user identification information as described above, it is difficult to map the items of information one to one.

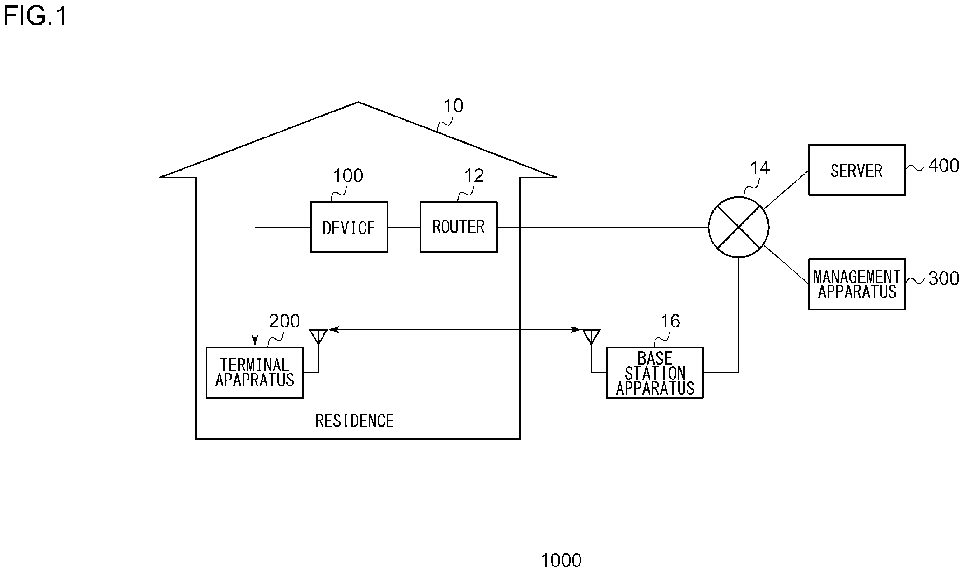

Fig. 1 shows a configuration of a communication system 1000. The communication system 1000 includes a router 12, a network 14, a base station apparatus 16, a device 100, a terminal apparatus 200, a management apparatus 300, and a server 400. The router 12, the device 100, and the terminal apparatus 200 are provided in a residence 10.

The device 100 is exemplified by an electric toilet seat, a delivery locker, and a residential elevator. It will be assumed here that the device 100 is an electric toilet seat. An electric toilet seat has a cleaning function, a heating function, etc. In particular, user-specific settings are possible for the cleaning function, the heating function, etc. The electric toilet seat is provided in the residence 10 so that the user in this case is a resident of the residence 10. Therefore, user settings for ten persons or fewer (e.g., four persons) are possible in the electric toilet seat. The device 100 has a communication function of ECHONET Lite (registered trademark), etc. and is connected to the router 12. Further, the router 12 is connected to the management apparatus 300 via the network 14. The network 14 is exemplified by the Internet.

The management apparatus 300 is a server for managing the functions of the electric toilet seat. The management apparatus 300 corresponds to the first system mentioned above and provides only the service for the functions of the electric toilet seat. A publicly known technology may be used for the service, and a description thereof is omitted. The user of each device 100 in the first system is identified by the device user identification information. User-specific settings mentioned above are realized by the device user identification information. Therefore, the total number of items of device user identification information for each device 100 is 10 or smaller (e.g., four). The management apparatus 300 may be comprised of a single apparatus or a plurality of apparatuses. The management apparatus 300 comprised of a plurality of apparatuses may be called a management system.

Meanwhile, the server 400, as well as the management apparatus 300, is connected to the network 14. The server 400 corresponds to the second system mentioned above and provides e-commerce service. The user in the second system is identified by the user identification information. In the case the second system provides e-commerce service, the total number of items of user identification information is 10,000 or larger. The first system manages the device 100, but the second system does not manage the device 100. The first system and the second system are systems independent of each other, and the device user identification information and the user identification information are not related to each other. Further, the total number of items of user identification information is larger than the total number of items of user identification information, and it is difficult to map them one to one. However, it is desirable to coordinate the first system and the second system by mapping the information to each other.

The terminal apparatus 200 is a wireless communication apparatus used by the user and is exemplified by a smartphone. The terminal apparatus 200 can be connected to the network 14 via the base station apparatus 16 by performing wireless communication with the base station apparatus 16. Wireless communication is exemplified by long term evolution (LTE) and wireless local area network (LAN). Therefore, the terminal apparatus 200 can benefit from the e-commerce service by accessing the server 400. Further, the terminal apparatus 200 can configure various functions of the electric toilet seat by accessing the management apparatus 300. Further, the terminal apparatus 200 includes a communication function for communicating with the device 100. The communication function is compatible with, for example, Bluetooth (registered trademark) or near field communication (NFC) .

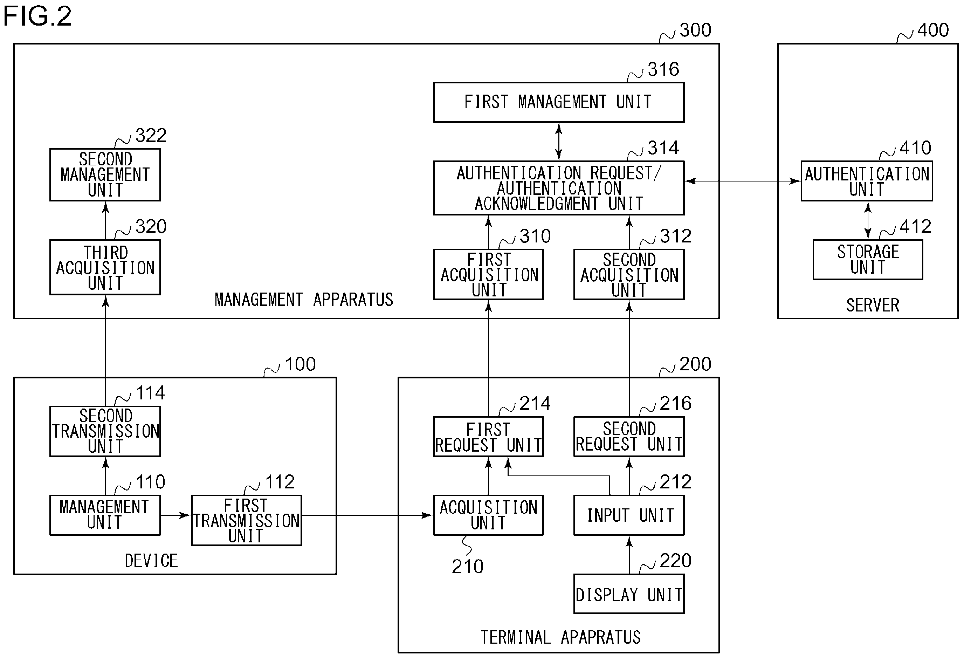

Fig. 2 shows functional blocks of the communication system 1000. Of the components of the communication system 1000 of Fig. 1, the device 100, the terminal apparatus 200, the management apparatus 300, and the server 400 are shown. The device 100 includes a management unit 110, a first transmission unit 112, a second transmission unit 114. The terminal apparatus 200 includes an acquisition unit 210, an input unit 212, a first request unit 214, a second request unit 216, and a display unit 220. The management apparatus 300 includes a first acquisition unit 310, a second acquisition unit 312, an authentication request/authentication acknowledgment unit 314, a first management unit 316, a third acquisition unit 320, and a second management unit 322. The server 400 includes an authentication unit 410 and a storage unit 412.

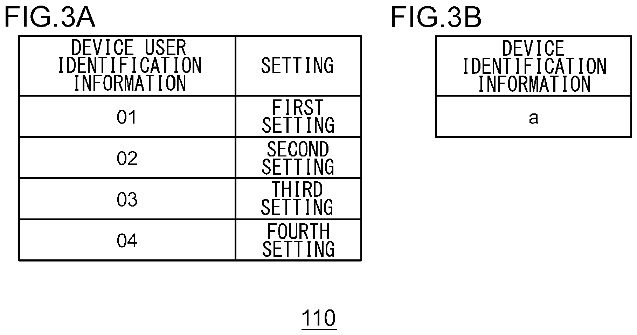

The management unit 110 of the device 100 manages information in the device 100. Figs. 3A-3B show a data structure of data stored in the management unit 110. Fig. 3A shows details of use-specific settings in the device 100. As mentioned above, the device user identification information is used for use-specific settings. By way of one example, "01" through "04" are used as the device user identification information, and settings for the respective users are shown as "first setting" through "fourth setting". In the case the device 100 is an electric toilet seat, "first setting" through "fourth setting" indicate the value of the amount of water used in cleaning, etc. These items of data are also used when the device 100 is not connected to the network 14 of Fig. 1. Meanwhile, Fig. 3B shows information (hereinafter, "device identification information") with which the management apparatus 300 identifies the device 100 connected to the network 14. By using the device identification information, the management apparatus 300 can uniquely identify a variety of devices 100 provided in the residence 10. Reference is made back to Fig. 2.

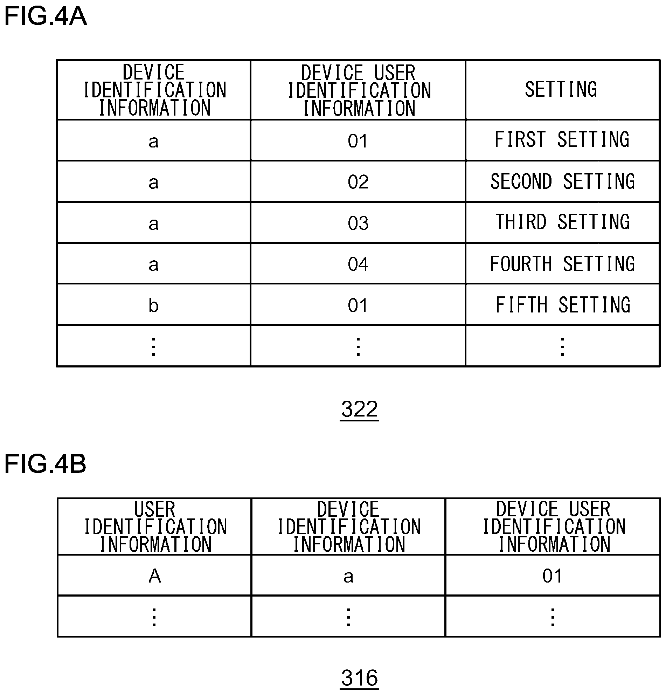

The second transmission unit 114 is connected to the router 12 of Fig. 1 and transmits the data stored in the management unit 110 to the management apparatus 300 via the router 12 and the network 14. The third acquisition unit 320 of the management apparatus 300 is connected to the network 14 and receives the data from the device 100. The second management unit 322 stores the data received in the third acquisition unit 320. Figs. 4A-4B show a data structure of data stored in the management apparatus 300. Fig. 4A shows data stored in the second management unit 322. As illustrated, the device identification information, the device user identification information, and setting are mapped to each other and are stored accordingly. The management apparatus 300 can provide only the service in the first system (e.g., the service for the functions of the electric toilet seat) by using the data stored in the second storage unit 322. Fig. 4B will be described later, and reference is made back to Fig. 2.

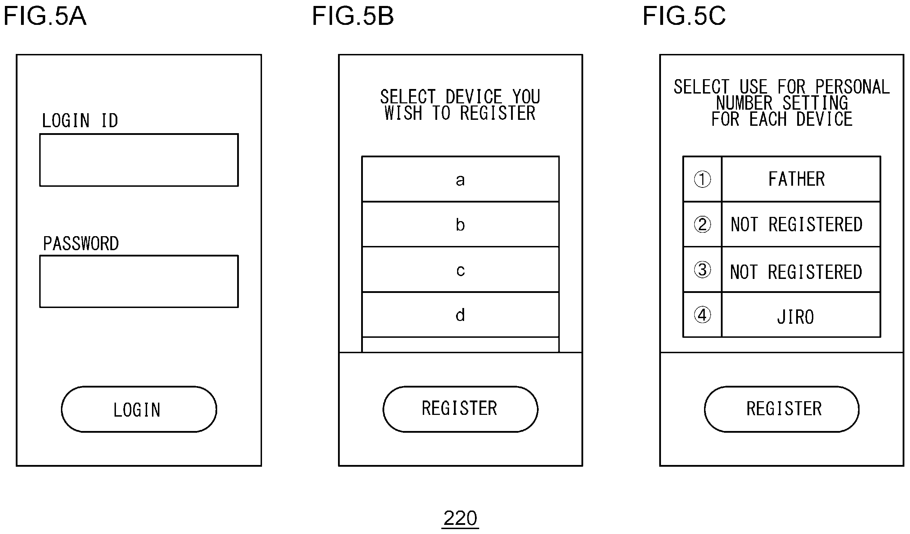

The display unit 220 of the terminal apparatus 200 displays certain information on a screen. The input unit 212 receives a user operation in the screen displayed on the display unit 220. The user operation is performed by using a button, etc. but may be performed by using a touch-sensitive panel integrated with the display unit 220. Figs. 5A-5C show screens displayed on the display unit 220. Fig. 5A shows a log-in screen for logging in the service provided by the server 400, i.e., the service in the second system. The user inputs user identification information as a login ID and also inputs a password. The input unit 212 acquires the user identification information and the password. For example, the user identification information is shown as "A", and the password is shown as "xxxxxxxx". Subsequently, the terminal apparatus 200 may log in the second system by communicating with the server 400 via the base station apparatus 16 and the network 14, but a description of the process thereof is omitted. Figs. 5B-5C will be described later, and reference is made back to Fig. 2.

The input unit 212 outputs the user identification information and the password received to the first request unit 214 and the second request unit 216. The user identification information and the password may be stored in the terminal apparatus 200 in advance. The user identification information and the password may be an access token issued after user authentication.

The user brings the terminal apparatus 200 close to the device 100. The first transmission unit 112 of the device 100 transmits the device identification information stored in the management unit 110, and the acquisition unit 210 of the terminal apparatus 200 receives the device identification information from the device 100. For example, the device identification information is indicated as "a". The acquisition unit 210 outputs the device identification information to the first request unit 214. Fig. 5B is a view of a screen showing a list of a plurality of devices 100.
The user selects, from the list, the device 100 corresponding to the device identification information "a" in the acquisition unit 210. The first request unit 214 requests the management apparatus 300 to register the device 100 by combining the device identification information "a", the user identification information "A", and the password "xxxxxxxx" and transmitting the combination to the management apparatus 300.

The first acquisition unit 310 of the management apparatus 300 receives a request for registration of the device 100 from the terminal apparatus 200 by receiving the combination of the the device identification information, the user identification information, and the password from the terminal apparatus 200. The first acquisition unit 310 outputs the combination of the device identification information, the user identification information, and the password to the authentication request/authentication acknowledgment unit 314. The authentication request/authentication acknowledgment unit 314 connects to the server 400 to request authentication (hereinafter, "first authentication") by the server 400 by using the user identification information and the password.

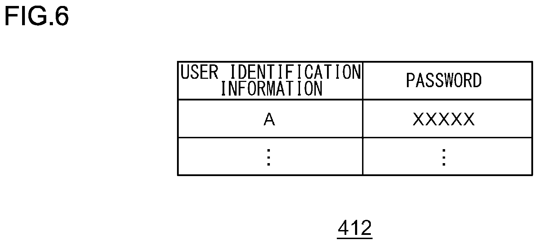

The authentication unit 410 of the server 400 is requested by the management apparatus 300 to perform the first authentication. The authentication unit 410 authenticates the user by using the data stored in the storage unit 412 as well as the user identification information and the password in the first authentication.
The storage unit 412 stores authentication information to authenticate the user. Fig. 6 shows a data structure of data stored in the storage unit 412. As illustrated, the combination of the user identification information and the password for the legitimate user is stored. Reference is made back to Fig. 2. The authentication unit 410 transmits the result of authentication, i.e., information indicating grant or rejection, to the management apparatus 300. The authentication request/authentication acknowledgment unit 314 receives the result of authentication from the server 400. When the result of authentication indicates that the first authentication is provided, the first management unit 316 stores the device identification information and the user identification information received in the first acquisition unit 310.

The display unit 220 of the terminal apparatus 200 displays a screen (hereinafter, "personal number input screen") for inputting information identifying the user using the device 100, i.e., the personal number. Fig. 5C shows a personal number input screen. In this case, a list of users using the device 100 is shown. The user uses the input unit 212 to select the user for which the setting in the device 100 is desired to be changed or the user for which the setting in the device 100 is desired to be checked. The input unit 212 outputs information related to the selected user to the second request unit 216. The information related to the selected user is the device user identification information mentioned above. For example, the device user identification information is shown as "01". The second request unit 216 requests the management apparatus 300 to register the user "01" for user registration, by combining the device user identification information "01", the user identification information "A", and the password "xxxxxxxx" and transmitting the combination to the management apparatus 300.

The second acquisition unit 312 of the management apparatus 300 receives a request for user registration of the user from the terminal apparatus 200 by receiving the combination of the the device user identification information, the user identification information, and the password from the terminal apparatus 200. As mentioned above, the total number of items of user identification information is larger than the total number of items of device user identification information. The second acquisition unit 312 outputs the combination of the device identification information, the user identification information, and the password to the authentication request/authentication acknowledgment unit 314. The authentication request/authentication acknowledgment unit 314 connects to the server 400 to request authentication (hereinafter, "second authentication") by the server 400 by using the user identification information and the password.

The authentication unit 410 of the server 400 is requested by the management apparatus 300 to perform the second authentication. The authentication unit 410 authenticates the user by using the data stored in the storage unit 412 as well as the user identification information and the password in the second authentication. For user authentication, the same process as that of the first authentication is performed. The authentication unit 410 transmits the result of authentication to the management apparatus 300.

The authentication request/authentication acknowledgment unit 314 of the management apparatus 300 receives the result of authentication from the server 400. When the result of authentication indicates that second authentication is provided, the first management unit 316 stores the device identification information and the user identification information received in the second acquisition unit 312. In the case the device identification information and the user identification information are already stored, the first management unit 316 maps the user identification information, the device identification information, and the device user identification information to each other, by using the user identification information. In other words, the first management unit 316 maps the user identification information "A", the device identification information "a", and the device user identification information "01" to each other and manage the information accordingly, by using the user identification information. The data stored in the first management unit 316 is as shown in Fig. 4B. The data linked to each other in this way may be stored in the storage unit 412 of the server 400.

The device, the system, or the entity that executes the method according to the disclosure is provided with a computer. By causing the computer to run a program, the function of the device, the system, or the entity that executes the method according to the disclosure is realized. The computer is comprised of a processor that operates in accordance with the program as a main hardware feature. The disclosure is non-limiting as to the type of the processor so long as the function is realized by running the program. The processor is comprised of one or a plurality of electronic circuits including a semiconductor integrated circuit (IC) or a large-scale integration (LSI). The plurality of electronic circuits may be integrated in one chip or provided in a plurality of chips. The plurality of chips may be aggregated in one device or provided in a plurality of devices. The program is recorded in a non-transitory recording medium such as a computer-readable ROM, optical disk, and hard disk drive. The program may be stored in a recording medium in advance or supplied to a recording medium via a wide area communication network including the Internet.

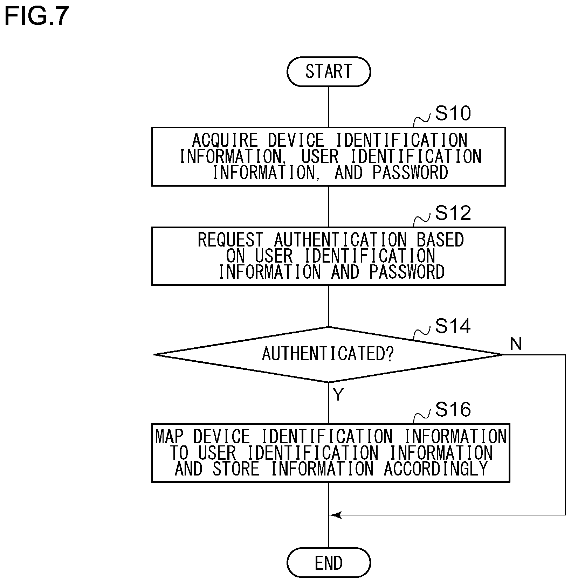

A description will be given of the operation of the communication system 1000 having the configuration described above. Fig. 7 is a flowchart showing a sequence of steps performed by the management apparatus 300. The first acquisition unit 310 acquires the device identification information, the user identification information, and the password (S10). The authentication request/authentication acknowledgment unit 314 asks the server 400 for authentication, based on the user identification information and the password (S12). When authentication is provided (Y in S14), the first management unit 316 maps the device identification information to the user identification information to each other and stores the information accordingly (S16). When authentication is not provided (N in S14), the process is terminated.

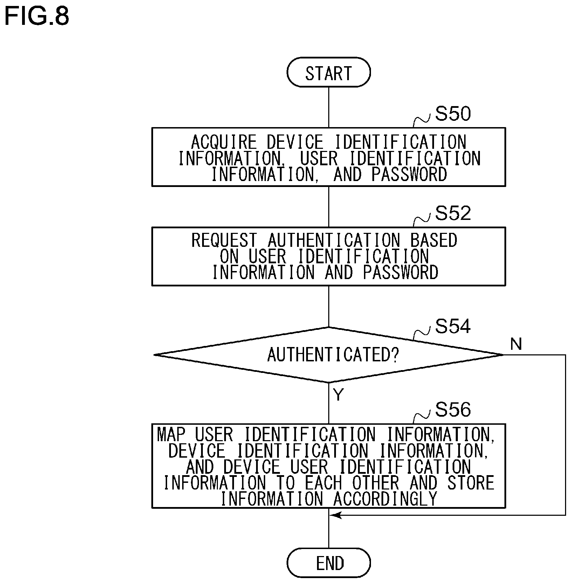

Fig. 8 is a flowchart showing another sequence of steps performed by the management apparatus 300. The second acquisition unit 312 acquires the device user identification information, the user identification information, and the password (S50). The authentication request/authentication acknowledgment unit 314 asks the server 400 for authentication based on the user identification information and the password (S52). When authentication is provided (Y in S54), the first management unit 316 maps the user identification information, the device identification information, and the device user identification information to each other and stores the information accordingly (S56). When authentication is not provided (N in S54), the process is terminated.

According to the embodiment, the user identification information, the device identification information, and the device user identification information are mapped to each other and are managed accordingly, by using the user identification information. It is therefore possible to coordinate the second system with the device 100. Further, since the user identification information, the device identification information, and the device user identification information are mapped to each other and are managed accordingly, the systems can refer to their information mutually. Further, since the systems can refer to their information mutually, the service quality is improved. Further, since the total number of items of user identification information is larger than the total number of items of device user identification information, different sets of information for which the total numbers differ can be mapped to each other. Further, since the device user identification information, the user identification information, and the password are used as well as the device user identification information, the user identification information, and the password, the user identification information and the device user identification information, for which the total numbers differ, can be mapped to each other.

One embodiment of the present invention is summarized below. A management apparatus 300 according to an embodiment of the disclosure includes: a first acquisition unit 310 that acquires device identification information for identifying a device 100 and user identification information for identifying a user in a system that does not manage the device 100; a second acquisition unit 312 that acquires device user identification information for identifying a user in the device 100 and the user identification information; and a first management unit 316 that maps the user identification information, the device identification information, and the device user identification information to each other and manages the information accordingly, by using the user identification information.

A total number of items of the user identification information that can be acquired by the first acquisition unit 310 and the second acquisition unit 312 is larger than a total number of items of the device user identification information that can be acquired by the second acquisition unit 312.

The apparatus may further include an authentication request/authentication acknowledgment unit 314 that connects to a server 400 of the system capable of authenticating a user based on the user identification information and a password corresponding to the user identification information. The first acquisition unit 310 may acquires the device identification information, the user identification information, and the password from a terminal apparatus 200 that acquires the device identification information from the device 100, the authentication request/authentication acknowledgment unit 314 may request the server 400 to perform first authentication by using the user identification information and the password acquired by the first acquisition unit 310, the second acquisition unit 312 may acquire the device user identification information, the user identification information, and the password from the terminal apparatus 200, the authentication request/authentication acknowledgment unit 314 may request the server 400 to perform second authentication by using the user identification information and the password acquired by the second acquisition unit 312, and when the first authentication is provided and the second authentication is provided, the first management unit 316 may map the user identification information, the device identification information, and the device user identification information to each other and manage the information accordingly.

Another embodiment of the present disclosure is a management method. The method includes: acquiring device identification information for identifying a device and user identification information for identifying a user in a system that does not manage the device; acquiring device user identification information for identifying a user in the device and the user identification information; and mapping the user identification information, the device identification information, and the device user identification information to each other and managing the information accordingly, by using the user identification information.

Described above is an explanation based on an exemplary embodiment. The embodiment is intended to be illustrative only and it will be understood by those skilled in the art that various modifications to constituting elements and processes could be developed and that such modifications are also within the scope of the present disclosure.

### [REFERENCE SIGNS LIST]

100 device, 110 management unit, 112 first transmission unit, 200 terminal apparatus, 210 acquisition unit, 212 input unit, 214 first request unit, 216 second request unit, 220 display unit, 300 management apparatus (management system), 310 first acquisition unit, 312 second acquisition unit, 314 authentication request/authentication acknowledgment unit, 316 first management unit (management unit), 400 server, 410 authentication unit, 412 storage unit, 1000 communication system

### [INDUSTRIAL APPLICABILITY]

According to the present disclosure, it is possible to coordinate a system different from the system that manages the device with the device.

## Claims

1. A management system comprising:
a first acquisition unit that acquires device identification information for identifying a device and user identification information for identifying a user in a system that does not manage the device;
a second acquisition unit that acquires device user identification information for identifying a user in the device and the user identification information; and
a management unit that maps the user identification information, the device identification information, and the device user identification information to each other and manages the information accordingly, by using the user identification information.

2. The management system according to claim 1, wherein a total number of items of the user identification information that can be acquired by the first acquisition unit and the second acquisition unit is larger than a total number of items of the device user identification information that can be acquired by the second acquisition unit.

3. The management system according to claim 1 or 2, further comprising:
a connection unit that connects to a server of the system capable of authenticating a user based on the user identification information and a password corresponding to the user identification information, wherein
the first acquisition unit acquires the device identification information, the user identification information, and the password from a terminal apparatus that acquires the device identification information from the device,
the connection unit requests the server to perform first authentication by using the user identification information and the password acquired by the first acquisition unit,
the second acquisition unit acquires the device user identification information, the user identification information, and the password from the terminal apparatus,
the connection unit requests the server to perform second authentication by using the user identification information and the password acquired by the second acquisition unit, and
when the first authentication is provided and the second authentication is provided, the management unit maps the user identification information, the device identification information, and the device user identification information to each other and manages the information accordingly.

4. A management method comprising:
acquiring device identification information for identifying a device and user identification information for identifying a user in a system that does not manage the device;
acquiring device user identification information for identifying a user in the device and the user identification information; and
mapping the user identification information, the device identification information, and the device user identification information to each other and managing the information accordingly, by using the user identification information.

5. A program that causes a computer to perform the management method according to claim 4.
